# EUROPEAN PATENT APPLICATION

(11) **EP 4 201 457 A1**
(43) Date of publication of application: **28.06.2023**
(21) Application number: 21216935.3
(22) Date of filing: 22.12.2021
(51) Int. Cl.: A61M 11/00, A61M 15/00, A61M 15/08

(54) **ATOMIZING MEANS**

(71) Applicant: Gerresheimer Regensburg GmbH, 93047 Regensburg (DE)
(72) Inventor: DEHLING, Michael, 92256 Hahnbach (DE)
(74) Representative: Bardehle Pagenberg Partnerschaft mbB Patentanwälte Rechtsanwälte

(57) **Abstract**

The present invention relates to an atomizing means 1, particularly for a nasal spray. The atomizing means comprises a syringe barrel 2 and an atomizer 3. The syringe barrel 2 comprises a first opening 4 and a second opening 5, wherein a first inner volume 15 is defined between the first opening 4 and the second opening 5. Thereby, the first opening 4 is configured for receiving a plunger 6. Further, the syringe barrel 2 comprises a syringe tip 7. The syringe tip 7 comprises a fluid inlet 8 and a fluid outlet 9, wherein a second inner volume 16 is defined between the fluid inlet 8 and the fluid outlet 9. The fluid inlet 8 is in communication with the second opening 5 of the syringe barrel 2. Moreover, the atomizer 3 is fixedly attached to the fluid outlet 9.

## Description

### Field of the invention

The present disclosure relates to an atomizing means, an atomizing system and a method for manufacturing an atomizing means.

### Background

For the treatment of various medical symptoms, atomized fluids which may comprise active ingredients are used. Particularly for treating respiratory diseases and/or their symptoms the application of an atomizing system, such as a nasal spray, a throat spray, an ear spray and/or an oral cavity spray, is widely known. Moreover, for the treatment of external injuries, such as open wounds and/or contusions, it is also practiced applying atomized fluids comprising active ingredients by means of atomizing systems.

Prior to atomizing a fluid, it is usually stored in a reservoir of the atomizing system. To atomize the stored fluid, a pressure is applied to at least a portion of the stored fluid, which is thereby urged through an atomizer, e.g. a nozzle, which atomizes the fluid.

A first group of atomizing systems comprises a spray head which is typically integrally formed with the atomizer, e.g. by means of injection molding. Thereby the spray head is attached to the reservoir storing the fluid. Said spray head can be pushed down towards the reservoir to urge a portion of the fluid through the atomizer. Due to this spray head actuation, said fluid portion is atomized. After the actuation (spray stroke) the spray head is released, and the spray head is pushed back to its initial position by an elastic element. Then, the atomizer is ready for a further spray stroke. The atomizing systems of the first group are also referred to as pumping atomizers and/or pump dispensers.

The amount of fluid being atomized by an atomizing system of the first group is defined and limited by the single spray stroke. Thus, it is only possible to empty the whole reservoir with multiple spray strokes. This is particularly problematic if a fast application of larger amounts of atomized fluid is required, exemplarily due to an emergency. Moreover, the dosage of an amount of fluid which lies between two spray strokes is difficult to realize.

A second group of atomizing systems comprises a syringe with a luer slip, wherein a spray head comprising an atomizer is plugged onto the luer slip prior to atomizing a fluid stored in the syringe. Using the syringe allows a continuous application of larger amounts of atomized fluid. Further, a better dosage may be achieved as a plunger of the syringe may be moved within a single stroke until a specific amount of atomized fluid is applied. This specific amount is not limited by a given spray stroke(s) as in the first group but can be set individually. Moreover, standardized syringes with luer slip can be processed and filled in well-known, large scale and stable processes.

However, the atomizing systems of the second group have the disadvantage that in case of an emergency which requires a fast application of the atomized fluid, the spray head must be plugged onto the luer slip prior to use. This plugged connection may get loose during the atomizing step and hence, active ingredients may be spilled. Further, the use of two separate parts which must be plugged together, is critical regarding sterility. Even further, providing separate parts to the end user increases the risk of incorrect assembly. Thus, there are pre-assembled systems. However, plugging the spray head onto the syringe at the end of the manufacturing and/or filling process, such as in in a pre-assembled system, is also problematic, particularly in terms of sealing such an atomizer system. Exemplarily, during transportation the spray head may be released. Moreover, when an external element is plugged onto the syringe, the syringe can no longer by processed, transported and/or filled in standardized and/or established processes. At least, those standardized processes have to be amended.

Thus, it is an object of the present disclosure to provide an atomizing means, an atomizing system and a method for manufacturing an atomizing means that overcome the aforementioned drawbacks at least partially.

### Description of the invention

These objects are achieved, at least partly, by an atomizing means, an atomizing system and a method for manufacturing an atomizing means, as defined in the independent claims. Further aspects of the present disclosure are defined in the dependent claims.

In particular, the object is achieved by an atomizing means, particularly for a nasal spray. The atomizing means may be configured for storing and/or atomizing a fluid, e.g. a fluid comprising an active ingredient. The active ingredient maybe an active pharmaceutical ingredient. Further, the fluid may comprise an antibiotic agent, an analgesic agent, an anticoagulant, a coagulant, a decongestant, an anticancer agent, an antirheumatic agent, an immunosuppressive agent, a hemostatic agent and/or a disinfectant.

The atomizing means comprises a syringe barrel and an atomizer. The syringe barrel may include a hollow cylinder. Moreover, the syringe barrel may comprise graduated marks which are configured to indicate a volume of fluid being stored inside the syringe barrel. Accordingly, a targeted dosing of the fluid comprising the active ingredient may be enhanced. The atomizer may be a liquid atomizer.

Further, the syringe barrel comprises a first opening and a second opening. The first and/or the second opening may have an annular shape. Moreover, the first opening may have a diameter which corresponds to an inner diameter of the hollow cylinder of the syringe barrel. Thus, fluid can be easily filled in the syringe barrel.

Moreover, a first inner volume is defined between the first opening and the second opening. The first opening is configured for receiving a plunger. Said plunger may comprise a seal which is configured such that when the plunger is displaced within the syringe barrel towards the syringe tip, a volume inside the syringe barrel is urged through the second opening. Accordingly, it is possible to dispense the fluid being stored inside the atomizing means with a plunger movement, particularly a continuous plunger movement. This is particularly advantageous for emergency applications which require a fast and/or a continuous application of an active ingredient. Moreover, this is also beneficial when a defined amount of an active ingredient is to be applied.

The syringe barrel further comprises a syringe tip. The syringe tip comprises a fluid inlet and a fluid outlet. Said fluid inlet and fluid outlet may define a second inner volume therebetween. The fluid inlet is in communication with the second opening of the syringe barrel. Hence, fluid can be transferred from the first inner volume to the fluid inlet and accordingly to the fluid outlet of the syringe tip.

The fluid inlet and the second opening may have the same diameter, wherein the fluid inlet and the second opening may be integrally formed. Even further the syringe tip may be integrally formed with the hollow cylinder of the syringe barrel, exemplarily by means of injection molding.

The atomizer is fixedly attached to the fluid outlet. Thus, a non-destructive separation of the atomizer and the fluid outlet may not be achieved. Further, the atomizer may be fixedly attached to the fluid outlet by means of gluing and/or welding. Preferably, the atomizer is attached to an exterior surface of the syringe tip. Particularly preferred, the atomizer is attached to a front face of the syringe tip. Thereby the shape of the syringe tip may not be changed for attaching the atomizer. Accordingly, the syringe tip may be easily equipped with an atomizer in few method steps.

The atomizing means may be configured for the application in any body orifice and/or for external body application. Particularly, the atomizing means may be for a throat spray, an ear spray, an oral cavity spray, an eye spray, a rectal spray, a vaginal spray and/or any other medical spray. Moreover, the atomizing means may be configured for the treatment of a rash, an external injury, such as an open wound and/or a contusion.

The syringe barrel and/or the atomizer may comprise a transparent material. Particularly, the syringe barrel and/or the atomizer may consist of a transparent material. Thus, a contamination and/or a fill level may be optically detected inside the syringe barrel and/or the atomizer. Further, the atomizer and/or the syringe barrel may comprise a cyclo-olefin polymer (COP), a glass material, a polypropylene polymer, a polyethylene polymer and/or any other sterilizable material. Moreover, the atomizer and/or the syringe barrel may consist of a cyclo-olefin polymer (COP), a glass material, a polypropylene polymer, a polyethylene polymer and/or any other sterilizable material. Particularly cyclo-olefin polymer (COP) is preferred, because of its high biocompatibility, in particular blood compatibility, as well as the low water absorption and water vapor permeability.

The atomizer may be welded to the fluid outlet, preferably by means of clear-to-clear welding. Clear-to-clear welding is also referred to as clear-to-clear laser welding and/or transparent laser plastic welding. Clear-to-clear welding is preferably used as it allows to weld transparent glass and/or polymer components with transparent glass and/or polymer components. The atomizer is preferably welded to the fluid outlet by means of at least one laser source which emits a laser with a wavelength from 1500 nm to 2500 nm, more preferably from 1600 nm to 2400 nm, even more preferably from 1700 nm to 2300 nm, and most preferably from 1750 nm to 2050 nm. Thereby the laser preferably at least partially penetrates the atomizer and/or the syringe tip.

Moreover, the atomizer may be welded to the fluid outlet by gas convection welding, heating element welding, infrared welding, laser welding, laser transmission welding, rotational friction welding, ultrasonic welding, vibration welding, hot gas welding, and/or circular welding.

The atomizer may be welded to the fluid outlet without a welding filler. Thus, a reaction of materials of the atomizer and/or the syringe barrel with the filler material may be prevented. Further, a reaction of the filler material with the fluid stored inside the atomizing means may be prevented. Accordingly, a risk of contamination is reduced.

The syringe tip may have a cylindrical shape or a conical shape. Particularly, the syringe tip may have a luer taper. General features of luer taper connectors are exemplarily defined in the standard DIN EN ISO 80369. Further particularly, the syringe tip may be a luer-cone which is optionally configured for a luer-slip connection. Moreover, an outer surface of the syringe tip may enclose an angle with a center axis from 1.5° to 2°, preferably from 1.6° to 1.9°, more preferably from 1.65° to 1.8°, even more preferably from 1.7° to 1.75°, and most preferably from 1.71° to 1.73°. The center axis may be a center axis of the atomizer, of the syringe tip and/or of the syringe barrel. Thus, the syringe barrel may comprise a standard syringe tip. Accordingly, the syringe barrel may be a standard syringe barrel which is commonly used in the medical field. Therefore, the atomizing means may be used, processed, transported and/or filled by established medical systems and/or methods.

The syringe tip may comprise a first through hole, wherein the first through hole preferably extends from the fluid inlet to the fluid outlet. The first through hole may have a cylindrical shape or a conical shape. It has to be noted that the shape of the through hole may change over its length. For example, a first section may be cylindrically shaped, and a second section may be tapered, i.e. conical. Preferably the first through hole at least partially forms part of the second inner volume. Moreover, the first through hole is preferably connected to the first inner volume.

The atomizer may have a cylindrical shape and/or a conical shape. Particularly an outer surface of the atomizer may have a cylindrical shape and/or a conical shape. Thereby the atomizer may have an axial length from 0.3 mm to 4 mm, preferably from 0.5 mm to 3.5 mm, more preferably from 0.7 mm to 3 mm, even more preferably from 0.8 mm to 2.5 mm, and most preferably from 1 mm to 2 mm. Further, the outer surface of the atomizer may enclose an angle with the center axis from 1.5° to 2°, preferably from 1.6° to 1.9°, more preferably from 1.65° to 1.8°, even more preferably from 1.7° to 1.75°, and most preferably from 1.71° to 1.73°.

The atomizer may comprise a second through hole, wherein the second through hole preferably has a diameter in a range from 0.05 mm to 1.5 mm, more preferably from 0.1 mm to 1.0 mm, even more preferably from 0.2 mm to 0.6 mm, and most preferably from 0.3 mm to 0.4 mm. Thereby the diameter maybe a maximum diameter or a minimum diameter of the second through hole. Further, the diameter may vary along the center axis. Further, the second through hole may have a cylindrical shape and/or a conical shape. The shape of the second through hole may change over its length. For example, a first section may be cylindrically shaped, and a second section may be tapered, i.e. conical.

Particularly, the maximum diameter of the second through hole d₂ may be smaller than the minimum diameter of the first through hole d₁. The ratio d₁:d₂ maybe in the range of 1.2 to 8, preferably in a range of 2 to 6 and most preferably in a range of 3 to 4.5.

Accordingly, the second through hole may be adapted to different fluids which are supposed to be atomized. Moreover, a specific atomizing behavior may be obtained. Exemplarily, the second through hole may be adapted to achieve a wide atomization or a narrow atomization. Further exemplarily, the second through hole may be adapted to achieve a fine atomization or a coarse atomization. Further, the atomizer may comprise multiple through holes.

The atomizing means may comprise a continuous transition from the outer surface of the syringe tip to the outer surface of the atomizer. Thereby the outer surface of the syringe tip and the outer surface of the atomizer may be connected. Thus, no gap may be formed between the outer surface of the syringe tip and the outer surface of the atomizer. Moreover, the outer surface of the syringe tip and the outer surface of the atomizer may form a continuous surface. Further, the continuous transition may include that an angle of the outer surface of the syringe tip corresponds to an angle of the outer surface of the atomizer. Thus, the continuous transition may include that no edge is formed between the outer surface of the syringe tip and the outer surface of the atomizer. This allows that connectors and/or caps for standard syringe tips such as luer cones may be applied to the atomizing means.

The atomizer may be configured to atomize a fluid which is urged through the atomizer in a direction from the fluid inlet to the fluid outlet. Thereby, atomization in the sense of the present invention may be understood as converting a volume of fluid into a plurality of droplets which form part of an aerosol. Parameters of atomization may be a size of the droplets, a contour of the droplet mist, a shape of the droplets and/or a speed of the droplets. Said parameters may be influenced by the geometry of the atomizer. Further, the parameters may be also influenced by a force which is applied to urge the fluid through the atomizer. Accordingly, the parameters of atomization may be actively influenced by the force which a user applies.

Furthermore, the atomizing means may comprise a sealing element for sealing the atomizing means. The sealing element may be a foil seal. Further, the sealing element may comprise or consist of cyclo-olefin polymer (COP). Even further, the sealing element maybe a self-bursting foil. Said self-bursting foil may burst at a predefined pressure being applied on the self-bursting foil. The sealing element may be applied on the outside of the atomizing means, on the second through hole of the atomizer. Further, the sealing element may be applied inside the syringe barrel on the second opening. The sealing element may be welded or glued to the syringe barrel and/or the atomizer, particularly the sealing element may be welded using a clear-to-clear welding technique.

Optionally, a contact element may be applied on an outer surface of the syringe tip and/or an outer surface of the atomizer. The contact element may have an outer shape which corresponds to a body orifice. Exemplarily, the outer shape of the contact element may correspond to the shape of a nostril and/or an ear canal. Preferably the contact element has a through hole and is slid over the syringe tip at least partially. The contact element maybe made of or comprise an elastic material, such as a silicone or a thermoplastic elastomer. The thermoplastic elastomer may be chosen from the group of styrenic block copolymers, TPS, thermoplastic polyolefinelastomers, TPO, thermoplastic vulcanizates, TPV, thermoplastic polyurethanes, TPU, thermoplastic copolyester, TPC and/or thermoplastic polyamides, TPA. The contact element allows to seal a gap between the atomizer and the patient's body. Hence, proper medication can be guaranteed.

Further, the object is at least partially achieved by an atomizing system comprising an atomizing means as described above, and a plunger which is configured for being inserted into the first opening. Preferably the plunger is inserted into the atomizing means. Moreover, the plunger may be axially movable inside the atomizing means.

The plunger may comprise a seal which is configured such that when the plunger is displaced within the syringe barrel towards the syringe tip, a volume inside the syringe barrel is urged through the second opening. Accordingly, it is possible to empty the atomizing system with a continuous movement of the plunger. This is particularly advantageous for emergency applications which require a fast application of an active ingredient. Further, a defined displacement of the plunger allows to provide a defined volume of liquid. Therefore, the atomizing system according to the present invention allows for a freely selectable and/or accurate dosage.

The syringe barrel may further comprise an inner storage volume storing an active ingredient. The inner storage volume may at least partially comprise the first inner volume and/or the second inner volume. Further, the inner storage volume may be changed, i.e. reduced or increased, by displacing the plunger inside the syringe barrel. Thereby the active ingredient may be an active pharmaceutical ingredient. Further, the active ingredient may comprise an antibiotic agent, an analgesic agent, an anticoagulant, a coagulant, a decongestant, an anticancer agent, an antirheumatic agent, an immunosuppressive agent, a hemostatic agent and/or a disinfectant.

Even further, the object is at least partially achieved by a method for manufacturing an atomizing means, wherein the atomizing means is preferably the atomizing means as described above. The method comprises at least the steps of
a. providing a syringe barrel comprising a first opening and a second opening, wherein a first inner volume is defined between the first opening and the second opening, wherein the first opening is configured for receiving a plunger, wherein the syringe barrel further comprises a syringe tip, wherein the syringe tip comprises a fluid inlet and a fluid outlet, wherein a second inner volume is defined between the fluid inlet and the fluid outlet, wherein the fluid inlet is in communication with the second opening of the syringe barrel;
b. providing an atomizer;
c. placing the atomizer on the fluid outlet, and
d. fixedly attaching the atomizer to the fluid outlet.

The atomizer may be welded to the fluid outlet, preferably by means of clear-to-clear welding. Thereby, the welding and optionally the clear-to-clear welding is preferably specified as described above.

### Brief description of the accompanying figures

In the following, the accompanying figures are briefly described:
- Fig. 1: is a schematic cut view of an atomizing means according to an embodiment of the present disclosure;
- Fig. 2: is a detailed schematic cut view of an exemplary atomizing means according to the present disclosure, wherein an exemplary syringe tip connected with an exemplary atomizer is depicted;
- Fig. 3: is another detailed schematic cut view of an exemplary atomizing means according to the present disclosure, wherein an exemplary syringe tip connected with an exemplary atomizer is depicted;
- Fig. 4: is a schematic cut view of an atomizing system according to the present disclosure;
- Fig. 5: is a further detailed schematic cut view of an exemplary atomizing means according to the present disclosure, wherein an exemplary syringe tip with an exemplary atomizer is depicted, wherein the process of welding the atomizer to the syringe tip is exemplarily illustrated, and
- Fig. 6: depicts a method for manufacturing an atomizing means.

### Detailed description of the figures

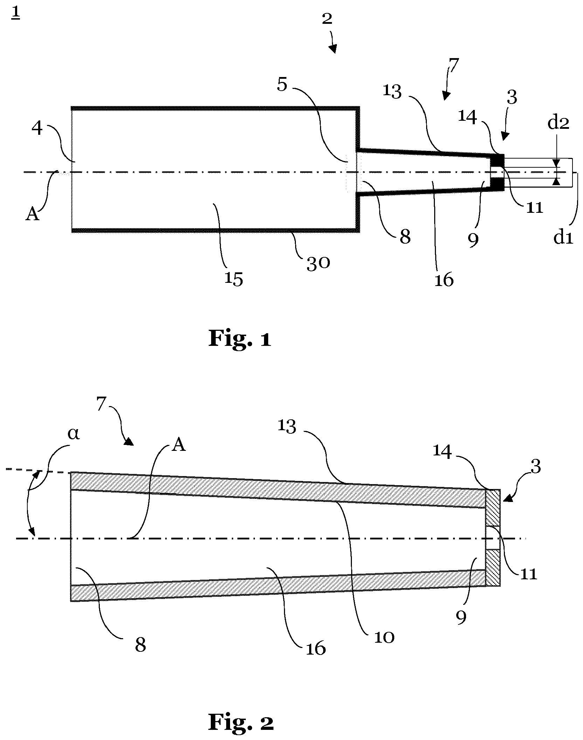

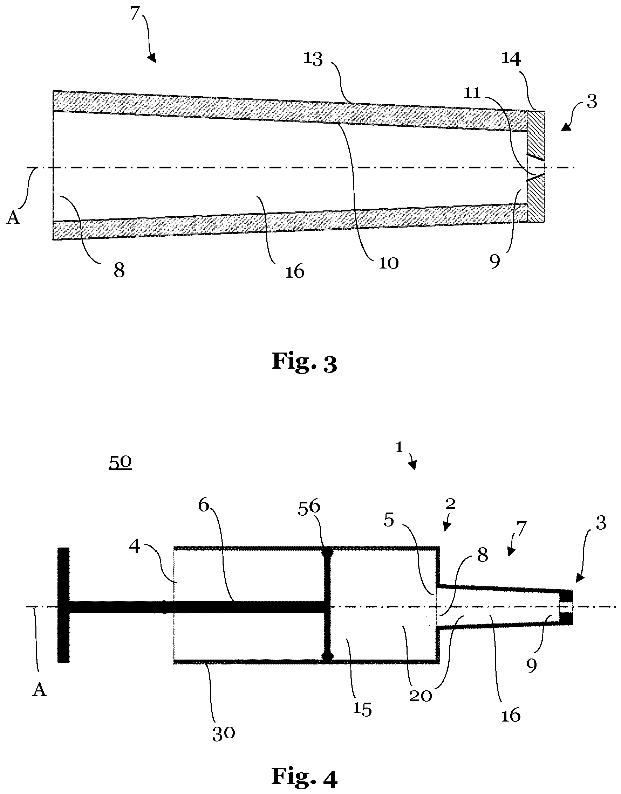

Fig. 1 is a schematic cut view of an atomizing means according to an embodiment of the present disclosure. The depicted atomizing means 1 comprises a syringe barrel 2 and an atomizer 3. The syringe barrel 2 comprises a first opening 4 and a second opening 5, wherein a first inner volume 15 is defined between the first opening 4 and the second opening 5. The first opening 4 is configured for receiving a plunger 6 (as e.g. depicted in Fig. 4). Moreover, the syringe barrel 2 comprises a syringe tip 7, wherein the syringe tip 7 comprises a fluid inlet 8 and a fluid outlet 9. A second inner volume 16 may be defined between the fluid inlet 8 and the fluid outlet 9. The fluid inlet 8 is in communication with the second opening 5 of the syringe barrel 2, and the atomizer 3 is fixedly attached to the fluid outlet 9.

In the embodiments shown in Figs. 1 to 5, a center axis A is illustrated. Thereby in Figs. 1 and 4 the center axis A is at least a center axis of the atomizer 3, of the syringe tip 7 and of the syringe barrel 2. Further, in Figs. 2, 3 and 5 the center axis A is at least a center axis of the atomizer 3 and of the syringe tip 7. In further embodiments the center axis A may be at least a center axis of the atomizer 3, of the syringe tip 7 and/or of the syringe barrel 2.

In the embodiment shown in Fig. 1 the syringe tip 7 is integrally formed with a hollow cylinder 30 of the syringe barrel 2. Moreover, the diameter of the first opening 4 corresponds to an inner diameter of the hollow cylinder 30.

The syringe tip 7 and the atomizer 3 depicted in Fig. 1 have a conical outer surface. Thereby the outer surface 14 of the atomizer 3 and/or the outer surface 13 of the syringe tip 7 each may enclose an angle with the center axis A from 1.5° to 2°, preferably from 1.6° to 1.9°, more preferably from 1.65° to 1.8°, even more preferably from 1.7° to 1.75°, and most preferably from 1.71° to 1.73°.

The syringe tip 7 comprises a first through hole 10. Thereby the first through hole 10 extends from the fluid inlet 8 to the fluid outlet 9. Moreover, the first through hole 10 has a conical shape.

The atomizer 3 comprises a second through hole 11 which has a cylindrical shape. Preferably the second through hole 11 has a diameter in a range from 0.05 mm to 1.5 mm, more preferably from 0.1 mm to 1.0 mm, even more preferably from 0.2 mm to 0.6 mm, and most preferably from 0.3 mm to 0.4 mm.

The atomizer 3 is configured to atomize a liquid which is urged through the atomizer 3 in a direction from the fluid inlet 8 to the fluid outlet 9.

In the embodiment depicted in Fig. 1, the atomizing means 1 comprises a continuous transition from an outer surface 13 of the syringe tip 7 to an outer surface 14 of the atomizer 3. Thereby the outer surface 13 of the syringe tip 7 and the outer surface 14 of the atomizer 3 are be connected. Further, no gap is formed between the outer surface 13 of the syringe tip 7 and the outer surface 14 of the atomizer 3. Moreover, the outer surface 13 of the syringe tip 7 and the outer surface 14 of the atomizer 3 form a continuous surface. Even further, the continuous transition includes that an angle of the outer surface 13 of the syringe tip 7 corresponds to an angle of the outer surface 14 of the atomizer 3. The continuous transition also includes that no edge is formed between the outer surface 13 of the syringe tip 7 and the outer surface 14 of the atomizer 3. This allows that connectors and/or caps for standard syringe tips such as luer cones may be applied to the atomizing means 1.

Fig. 2 is a detailed schematic cut view of an exemplary atomizing means 1 according to the present disclosure, wherein an exemplary syringe tip 7 connected with an exemplary atomizer 3 is depicted. The depicted detailed view discloses that the atomizing means 1 comprises a continuous transition from an outer surface 13 of the syringe tip 7 to an outer surface 14 of the atomizer 3. Thereby the outer surface 13 of the syringe tip 7 and the outer surface 14 of the atomizer 3 are be connected. Moreover, no gap is formed between the outer surface 13 of the syringe tip 7 and the outer surface 14 of the atomizer 3.

Further, as illustrated in Fig. 2, the first through hole 10 of the syringe tip 7 comprises a conical shape, wherein the second through hole 11 of the atomizer 3 comprises a cylindrical shape.

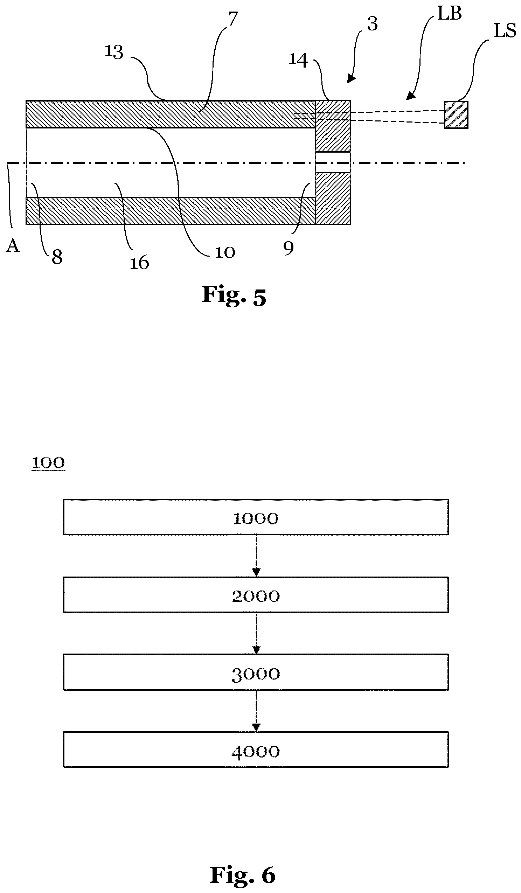

Moreover, as depicted by Fig. 2, but also Figs. 3 and 5, the atomizer 3 is attached to an exterior surface of the syringe tip 7. Thereby the shape of the syringe tip 7 does not necessarily need to be changed for attaching the atomizer 3. Accordingly, syringe tips may be easily equipped with an atomizer in few method steps.

The syringe tip 7 depicted in Fig. 2 has a conical outer surface 13. Thereby the outer surface 13 of the syringe tip 7 may enclose an angle a with the center axis A from 1.5° to 2°, preferably from 1.6° to 1.9°, more preferably from 1.65° to 1.8°, even more preferably from 1.7° to 1.75°, and most preferably from 1.71° to 1.73°.

Fig. 3 is another detailed schematic cut view of an exemplary atomizing means 1 according to the present disclosure, wherein an exemplary syringe tip 7 connected with an exemplary atomizer 3 is depicted. In the depicted embodiment the second through hole 11 has a conical shape. Accordingly, the diameter of the second through hole 11 varies along the center axis A. Accordingly, a specific atomizing behavior may be obtained. Exemplarily, the second through hole 11 may be adapted to achieve a wide atomization or a narrow atomization. Further exemplarily, the second through hole 11 may be adapted to achieve a fine atomization or a coarse atomization.

Fig. 4 is a schematic cut view of an atomizing system 50 according to the present disclosure. The depicted atomizing system 50 comprises the atomizing means 1 which is depicted in Fig. 1, and a plunger 6 which is inserted into the first opening 4. The plunger comprises a seal 56 which is configured such that when the plunger 6 is displaced within the syringe barrel 2 towards the syringe tip 7, a volume inside the syringe barrel 2 is urged through the second opening 5. Accordingly, it is possible to empty the atomizing system 50 at least partially with a continuous movement of the plunger 6. This is particularly advantageous for emergency applications which require a fast application of an active ingredient stored inside the atomizing system 50. Further, a defined displacement of the plunger 6 allows to provide a defined volume of liquid.

The syringe barrel 2 further comprises an inner storage volume 20 storing an active ingredient. The inner storage volume 20 partially comprises the first inner volume 15 and the second inner volume 16. Further, the inner storage volume 20 can be changed by axially displacing the plunger 6 inside the syringe barrel 2.

Fig. 5 is a further detailed schematic cut view of an exemplary atomizing means 1 according to the present disclosure, wherein an exemplary syringe tip 7 with an exemplary atomizer 3 is depicted, wherein the process of welding the atomizer 3 to the syringe tip 7 is exemplarily illustrated. The syringe tip 7 of the syringe barrel 2 and the atomizer 3 consist of a transparent material. Thus, a contamination maybe optically detected inside the syringe tip 7 and/or the atomizer 3. Preferably, the atomizer 3 and/or the syringe tip 7 may consist of a cyclo-olefin polymer (COP), a glass material, a polypropylene polymer, a polyethylene polymer and/or any other sterilizable material.

Moreover, Fig. 5 particularly illustrates clear-to-clear welding. Thereby, as depicted, a laser source LS emits a laser comprising laser beams LB. The laser preferably has a wavelength from 1500 nm to 2500 nm, more preferably from 1600 nm to 2400 nm, even more preferably from 1700 nm to 2300 nm, and most preferably from 1750 nm to 2050 nm. Moreover, the laser beams LB partially penetrate the atomizer and the syringe tip.

Further as depicted in Fig. 5, the atomizer 3 is welded to the fluid outlet 9 without a welding filler. Thus, a reaction of the materials of the atomizer 3 and/or the syringe barrel 2 with the filler material may be prevented. Further, a reaction of the filler material with the fluid stored inside the atomizing means 1 may be prevented. Accordingly, a risk of contamination is reduced.

Fig. 6 depicts a method 100 for manufacturing an atomizing means 1. Thereby the atomizing means 1 is preferably the atomizing means 1 as described with reference to the previous figures. The method 100 comprises at least the steps of
a. providing 1000 a syringe barrel 2 comprising a first opening 4 and a second opening 5, wherein a first inner volume 15 is defined between the first opening 4 and the second opening 5, wherein the first opening 4 is configured for receiving a plunger 6, wherein the syringe barrel 2 further comprises a syringe tip 7, wherein the syringe tip 7 comprises a fluid inlet 8 and a fluid outlet 9, wherein a second inner volume 16 is defined between the fluid inlet 8 and the fluid outlet 9, wherein the fluid inlet 8 is in communication with the second opening 5 of the syringe barrel 2;
b. providing 2000 an atomizer 3;
c. placing 3000 the atomizer 3 on the fluid outlet 9, and
d. fixedly attaching 4000 the atomizer 3 to the fluid outlet 9.

### List of reference signs

- 1: atomizing means
- 2: syringe barrel
- 3: atomizer
- 4: first opening
- 5: second opening
- 6: plunger
- 7: syringe tip
- 8: fluid inlet
- 9: fluid outlet
- 10: first through hole
- 11: second through hole

- 13: outer surface of the syringe tip 7
- 14: outer surface of the atomizer 3
- 15: first inner volume
- 16: second inner volume

- 20: inner storage volume
- 30: hollow cylinder
- 50: atomizing system
- 56: seal
- A: center axis
- α: angle
- LS: laser source
- LB: laser beams
- 100: method for manufacturing an atomizing means
- 1000: providing a syringe barrel
- 2000: providing an atomizer
- 3000: placing the atomizer
- 4000: fixedly attaching the atomizer

## Claims

1. An atomizing means (1), particularly for a nasal spray, comprising
a syringe barrel (2) and an atomizer (3), wherein
the syringe barrel (2) comprises a first opening (4) and a second opening (5), wherein a first inner volume (15) is defined between the first opening (4) and the second opening (5), and wherein the first opening (4) is configured for receiving a plunger (6), wherein
the syringe barrel (2) further comprises
a syringe tip (7), the syringe tip (7) including a fluid inlet (8) and a fluid outlet (9),
wherein the fluid inlet (8) is in communication with the second opening (5) of the syringe barrel (2), and wherein the atomizer (3) is fixedly attached to the fluid outlet (9).

2. The atomizing means (1) according to claim 1, wherein the syringe barrel (2) and/or the atomizer (3) comprise a transparent material, such as cyclic olefin copolymer, COP.

3. The atomizing means (1) according to any one of the preceding claims, wherein the atomizer (3) is welded to the fluid outlet (9), particularly by means of clear-to-clear welding.

4. The atomizing means (1) according to the preceding claim, wherein the atomizer (3) is welded to the fluid outlet (9) without a welding filler.

5. The atomizing means (1) according to any one of the preceding claims, wherein the syringe tip (7) has a cylindrical shape or a conical shape.

6. The atomizing means (1) according to any one of the preceding claims, wherein the syringe tip (7) comprises a first through hole (10), wherein the first through hole (10) may extend from the fluid inlet (8) to the fluid outlet (9).

7. The atomizing means (1) according to any one of the preceding claims, wherein the atomizer (3) has a cylindrical shape or a conical shape.

8. The atomizing means (1) according to any one of the preceding claims, wherein the atomizer (3) comprises a second through hole (11), wherein the second through hole (11) may have a diameter in a range from 0.05 mm to 1.5 mm, more preferably from 0.1 mm to 1.0 mm, even more preferably from 0.2 mm to 0.6 mm, and most preferably from 0.3 mm to 0.4 mm.

9. The atomizing means (1) according to any one of the preceding claims, wherein the atomizing means (1) comprises a continuous transition from an outer surface (13) of the syringe tip (7) to an outer surface (14) of the atomizer (3).

10. The atomizing means (1) according to any one of the preceding claims, wherein the atomizer (3) is configured to atomize a liquid which is urged through the atomizer (3) in a direction from the fluid inlet (8) to the fluid outlet (9).

11. An atomizing system (50) comprising
an atomizing means (1) according to any one of claims 1 to 10, and
a plunger (6) which is configured for being inserted into the first opening (4).

12. The atomizing system (50) according to claim 11, wherein the plunger (6) comprises a seal (56) which is configured such that when the plunger (6) is displaced within the syringe barrel (2) towards the syringe tip (7), a volume inside the syringe barrel (2) is urged through the second opening (5).

13. The atomizing system (50) according to any one of the preceding claims, wherein the syringe barrel (2) further comprises an inner storage volume (20) storing an active ingredient.

14. A method (100) for manufacturing an atomizing means (1), wherein the atomizing means (1) may be the atomizing means (1) according to any one of claims 1 to 10, wherein the method (100) comprises at least the steps of
a. providing (1000) a syringe barrel (2) comprising a first opening (4) and a second opening (5), wherein a first inner volume (15) is defined between the first opening (4) and the second opening (5), wherein the first opening (4) is configured for receiving a plunger (6), wherein the syringe barrel (2) further comprises a syringe tip (7), wherein the syringe tip (7) comprises a fluid inlet (8) and a fluid outlet (9), wherein the fluid inlet (8) is in communication with the second opening (5) of the syringe barrel (2);
b. providing (2000) an atomizer (3);
c. placing (3000) the atomizer (3) on the fluid outlet (9), and
d. fixedly attaching (4000) the atomizer (3) to the fluid outlet (9).

15. The method (100) according to the preceding claim, wherein the atomizer (3) is welded to the fluid outlet (9), particularly by means of clear-to-clear welding.
